# EUROPEAN PATENT APPLICATION

(11) **EP 1 466 592 A1**
(43) Date of publication of application: **13.10.2004**
(21) Application number: 04008206.7
(22) Date of filing: 05.04.2004
(51) Int. Cl.: A61K 7/50

(54) **Cleansing compositions**

(30) Priority: 07.04.2003 JP 2003102544
(71) Applicant: Kao Corporation, Tokyo 103-8210 (JP)
(72) Inventor: Sakurai, Naoe, c/o Kao Corp. Res. Lab., Tokyo 131-8501 (JP)
(74) Representative: HOFFMANN - EITLE

(57) **Abstract**

The present invention relates to a cleansing composition containing (A) a surfactant, (B) an amino acid or amphoteric polymer and (c) water and having a pH not greater than an isoelectric point of component (B) when diluted to 5 wt.% with purified water. Washing with the cleansing composition leaves substantially no taut feeling but moist feeling on the skin, while the hair shampooed with it is soft to the touch and is substantially free from tangles so that fingers glide through the hair smoothly.

## Description

### Field of the Invention

The present invention relates to cleansing compositions and a body cleansing method using the same.

### Background of the Invention

For cleansing compositions applied to the skin or hair, anionic surfactants such as alkyl sulfate ester salts, alkyl ether sulfate salts and α-olefin sulfonate salts have conventionally been employed widely as foaming surfactants. These surfactants have high cleansing power and provide a refreshed feeling after washing, but involve problems such as causing a taut feeling of the skin. As the so-called low-irritation surfactants, sulfosuccinic acid surfactants, ether carboxylic acid surfactants, amide ether carboxylic acid surfactants, N-alkylamide alkanol sulfate ester salts and the like have been proposed. Single use of these surfactants, however, is not satisfactory from the viewpoints of foaming property and foam quality.

A skin cleansing composition using an anionic surfactant and trimethylglycine in combination at a specific ratio is proposed as a cleansing composition with good foaming property and excellent feeling upon use (Japanese Patent Application Laid-Open No. Hei 11-180855). In spite of their improved foamability and foaming quality, the above-described composition is not satisfactory because a taut feeling or dry and rough feeling remains on the skin or hair after washing with it.

### Summary of the Invention

The present invention provides a cleansing composition containing (A) a surfactant, (B) an amino acid or amphoteric polymer, and (c) water, the composition having a pH not greater than an isoelectric point of component (B) when diluted to 5 wt.% with purified water.

The present invention also provides a cleansing composition containing (A) a surfactant, (B) an amino acid or amphoteric polymer, and (c) water, the composition having a pH not greater than an isoelectric point of component (B) when diluted with water upon use.

The present invention further provides a body cleansing method, which includes adding water to a cleansing composition comprising (A) a surfactant, (B) an amino acid or amphoteric polymer, and (c) water, the composition having a pH not greater than an isoelectric point of component (B) when diluted to 5 wt.% with purified water, and then washing a body with the resulting mixture.

### Detailed Description of the Invention

The present invention relates to a cleansing composition which has benefits including excellent stability, gives the skin a moist feeling after washing, causes neither a taut feeling nor a dry and rough feeling, makes the hair substantially tangle-free upon shampooing, does not give the hair a dry and rough feeling after shampooing. The present invention also relates to a body cleansing method using the cleansing composition.

The present inventor has found that the above-described problems can be overcome by using an aqueous cleansing composition containing a surfactant, and an amino acid or amphoteric polymer and selecting conditions so that the pH of the composition, when diluted with water upon use, does not exceed an isoelectric point of the amino acid or amphoteric polymer.

The surfactant to be used as the component (A) in the present invention may be any one of anionic surfactants, nonionic surfactants, amphoteric surfactants and cationic surfactants insofar as it has foaming properties suited for use as a body cleansing composition.

Examples of the anionic surfactant include polyoxyalkylene alkyl (or alkenyl) ether sulfates, alkyl (or alkenyl) sulfates, higher fatty acid salts, ether carboxylates, amide ether carboxylates, alkyl phosphate ester salts, N-acylamino acid salts, polyoxyalkylene fatty acid amide ether sulfates, acyl isethionates, and acyl taurates. Of these, alkyl phosphate ester salts, polyoxyethylene alkyl ether sulfates and N-acyl glutamates are preferred in that a composition containing such an anionic surfactant can be readily prepared as a weakly acidic cleanser.

Examples of the nonionic surfactant include amine oxides, glycerin fatty acid esters, sorbitan fatty acid esters, alkyl saccharides, polyoxyalkylene alkyl ethers, fatty acid alkanolamides, and polyoxyalkylene hydrogenated castor oils. Specifically, lauryl dimethylamine oxide, myristyl dimethylamine oxide, isostearic acid monoglyceride, oleic acid monoglyceride, sorbitan monolaurate, sorbitan monooleate, sorbitan sesquioleate, sorbitan monoisostearate, sorbitan trioleate, alkyl polyglucoside, coconut oil fatty acid monoethanolamide and coconut oil fatty acid diethanolamide are preferred.

Examples of the amphoteric surfactant include carbobetaines, sulfobetaines, imidazolinium betaines and amidobetaines. Of these, fatty acid amidopropylbetaine, hydroxypropyl sulfobetaine and desalted secondary imidazolinium betaine are preferred.

Examples of the cationic surfactant include mono- or di-alkyl quaternary ammonium salts which have a linear or branched alkyl group and may be added with an alkylene oxide. Of these, linear monoalkyl quaternary ammonium salts having from 12 to 16 carbon atoms, and quaternary ammonium salts with a branched alkyl group having from 20 to 28 carbon atoms are preferred.

As the component (A), the above-described anionic surfactants are preferred.

As the component (A), one or more surfactants may be used. In consideration of its good foaming properties and feeling after cleansing, the component (A) is preferably used in an amount of from 1 to 50 wt.%, more preferably from 3 to 30 wt.% based on the whole composition.

In the present invention, the above-described surfactant is used in combination with an amino acid and/or an amphoteric polymer both having amphoteric properties. These components are selected so that the pH of the resulting composition, when diluted with water upon use, does not exceed an isoelectric point of the amino acid or amphoteric polymer used. The term "isoelectric point" as used herein means a hydrogen ion concentration of an aqueous solution of the amphoteric substance at which the positive and negative charges in the molecule are balanced, that is, the net charge equals zero. It is a value measured at 25°C.

Examples of the amino acid used as the component (B) in the present invention include glycine (isoelectric point: 5.97), alanine (isoelectric point: 6.00), serine (isoelectric point: 5.68), arginine (isoelectric point: 10.8), histidine (isoelectric point: 7.47), threonine (isoelectric point: 5.64), asparagine (isoelectric point: 5.41), glutamine (isoelectric point: 5.65), leucine (isoelectric point: 5.98), valine (isoelectric point: 5.96), lysine (isoelectric point: 9.59), phenylalanine (isoelectric point: 5.48), tyrosine (isoelectric point: 5.66), urocanic acid (isoelectric point: 4.60), proline (isoelectric point: 6.30), oxyproline (isoelectric point: 5.74), isoleucine (isoelectric point: 5.94), cysteine (isoelectric point: 5.02), methionine (isoelectric point: 5.74), tryptophan (isoelectric point: 5.89), glutamic acid (isoelectric point: 3.22), and aspartic acid (isoelectric point: 2.77).

Examples of the amphoteric polymer include acrylic acid/dimethyldiallylammonium chloride copolymer ("MERQUAT 280": isoelectric point of 14 or greater, "MERQUAT 295": isoelectric point of 14 or greater, each product of ONDEO Nalco), acrylamide/acrylic acid/dimethyldiallylammonium chloride copolymer ("MERQUAT PLUS3330": isoelectric point: 5.0, "MERQUAT PLUS3331": isoelectric point of 7.0 or greater, "MERQUAT 3333": isoelectric point: 4.3, each product of ONDEO Nalco), methyl acrylate/acrylic acid/methacrylamidopropyltrimethylammonium chloride copolymer ("MERQUAT 2001": isoelectric point of 7.0, product of ONDEO Nalco), and acrylamide/acrylic acid/methacrylamidopropyltrimethylammonium chloride copolymers ("MERQUAT2003": isoelectric point of 7.0 or greater, product of ONDEO Nalco). Of these, acrylic acid/dimethyldiallylammonium chloride copolymer ("MERQUAT 280", "MERQUAT 295"), acrylamide/acrylic acid/dimethyldiallylammonium chloride copolymer ("MERQUAT PLUS3330", "MERQUAT PLUS3331"), and methyl acrylate/acrylic acid/methacrylamidopropyltrimethylammonium chloride copolymer ("MERQUAT 2001") are preferred.

As the component (B), those having an isoelectric point of from 3 to 12 are preferred, more preferably from 4 to 10, because of excellent properties of the resulting cleansing composition.

As the component (B), one or more compounds selected from amino acids and amphoteric polymers can be used. Its content preferably ranges from 0.1 to 30 wt.%, more preferably from 1 to 20 wt.% based on the whole composition, because of substantially no sticky feeling upon use.

In the cleansing composition of the present invention, water is preferably used in an amount of from 20 to 99 wt.%, more preferably from 50 to 96 wt.%, more preferably from 70 to 90 wt.% based on the whole composition.

When diluted with purified water to 5 wt.% at 25°C, or diluted with water upon use, the cleansing composition of the present invention has a pH not greater than the isoelectric point of the component (B). When two or more compounds are used as the component (B), the pH of the cleansing composition is adjusted so as not to exceed the isoelectric point of each of them. When the pH exceeds the isoelectric point of the component (B), the skin after washing has a taut feeling and cannot gain a sufficient moist feeling.

The pH of the composition may be adjusted with an aqueous acid or alkali solution.

Examples of the acid include inorganic acids such as hydrochloric acid, sulfuric acid and phosphoric acid and organic acids such as ascorbic acid, adipic acid, succinic acid, acetic acid, oxalic acid, lactic acid, malic acid, aspartic acid, and glutamic acid.

Examples of the alkali include sodium hydroxide, potassium hydroxide and basic amino acids such as arginine and lysine.

The cleansing composition of the present invention preferably has a pH at 25°C, when diluted with purified water to 5 wt.% or diluted with water upon use each, of 2 or greater but less than 11, more preferably from 4 to 10.

The pH of the diluted aqueous solution as mentioned above may be measured by a pH meter at 25°C.

When the pH of the aqueous solution is not greater than the isoelectric point, the amphoteric substance is positively charged. Although not wanting to be limited by theory, because the surface of the skin or hair usually stays negative, the amphoteric substance, which is positively charged under a pH not exceeding its isoelectric point upon use, tends to be adsorbed onto the skin or hair. As a result, the effect of the amphoteric substance can be obtained at a greater level.

The cleansing composition of the present invention may contain further components. Examples include lanolin and its derivatives, esters such as isopropyl myristate, oily components such as coconut oil triglycerides, humectants such as polyhydric alcohols and polyglyceryl fatty acid esters, bactericides such as triclosan and trichlorocarbanilide, anti-inflammatory agents such as potassium glycyrrhizinate and tocopherol acetate, antiseptics such as methylparaben, ethylparaben, propylparaben, butylparaben, sodium benzoate and isopropyl methyl phenol, chelating agents such as ethylenediaminetetraacetic acid or its salts and hydroxyethanediphosphonic acid or its salts, thickeners such as carboxyvinyl polymer, carrageenan, hydroxyethyl cellulose and cationic cellulose, salts such as sodium chloride, pearling agents, scrub agents, perfumes, cooling agent such as 1-menthol, coloring matters, UV absorbers, antioxidants and plant extracts.

The cleansing composition of the present invention can be prepared by mixing the above-described components. It can be provided, for example, as a skin cleansing composition such as a face wash, makeup cleanser, body shampoo and hand soap; and as a hair cleansing composition such as shampoo.

In the present invention, the pH of the cleansing composition is adjusted so as not to exceed the isoelectric point of the component (B) when the body, face, hands, feet or hair is washed with the composition added with water. The skin washed with the composition is therefore substantially free from a taut feeling and has a moist feeling, while the hair shampooed with the composition is soft to the touch and is substantially free from tangles so that fingers glide through the hair smoothly.

The following examples further describe and demonstrate embodiments of the present invention. The examples are given solely for the purpose of illustration and are not to be construed as limitations of the present invention.

### Examples

### Examples 1 to 4, Comparative Examples 1 to 4

Cleansing compositions as shown in Table 1 were prepared and evaluation were made in view of the existence of a taut feeling and the skin moisture content, each after washing with the composition, and the stability of the compositions. The amount of water is adjusted as a balance (to give a total amount of 100). The results are shown in Table 1.

In each Example, the pH of the composition was measured at 25°C by "Horiba pH meter F-22" (manufactured by Horiba, Ltd.) on a 5 wt.% aqueous solution obtained by diluting each composition with purified water.

### (Evaluation method)

### (1) Taut feeling after washing the skin

A panel of 10 Japanese female experts in their 20's or 30's foamed 1 g of each cleansing composition, massaged the whole face with the foam for 60 seconds, rinsed the face with water, and then towel dried their face. In accordance with the below-described criteria, they organoleptically evaluated the existence of a taut feeling of the skin. An average score was calculated. The composition having an average score of from 2.5 to 3.0 was ranked as A, that having an average score of from 1.5 to 2.4 was ranked as B, and that having an average score of from 1.0 to 1.4 was ranked as C.
3: No taut feeling
2: A little taut feeling
1: A strong taut feeling

### (2) Skin moisture content after washing

After washing the whole face as in evaluation method(1) and towel drying the face, a skin moisture content five minutes after towel drying was measured using the "Corneometer CM825" (manufactured by Courage + Khazaka electronic GmbH). The skin moisture content after washing was compared with that before washing and a moisture content ratio, after washing/before washing, was calculated. Each composition was evaluated in accordance with the below-described criteria.
A: The moisture content before and after face washing is almost equal (the moisture content ratio is 0.9 or greater).
B: The moisture content lowers compared with that before face washing (the moisture content ratio is 0.6 or greater but less than 0.9)
C: The moisture content greatly lowers compared with that before face washing (the moisture content ratio is less than 0.6)

### (3) Stability

Into a 100 mL glass sample bottle, 80 g of each cleansing composition was poured. After storage for 1 day at 25°C, its viscosity was measured and evaluated in accordance with the following criteria:
A: Almost no thickening has occurred.
B: Obvious thickening has occurred.
C: Marked thickening has occurred.

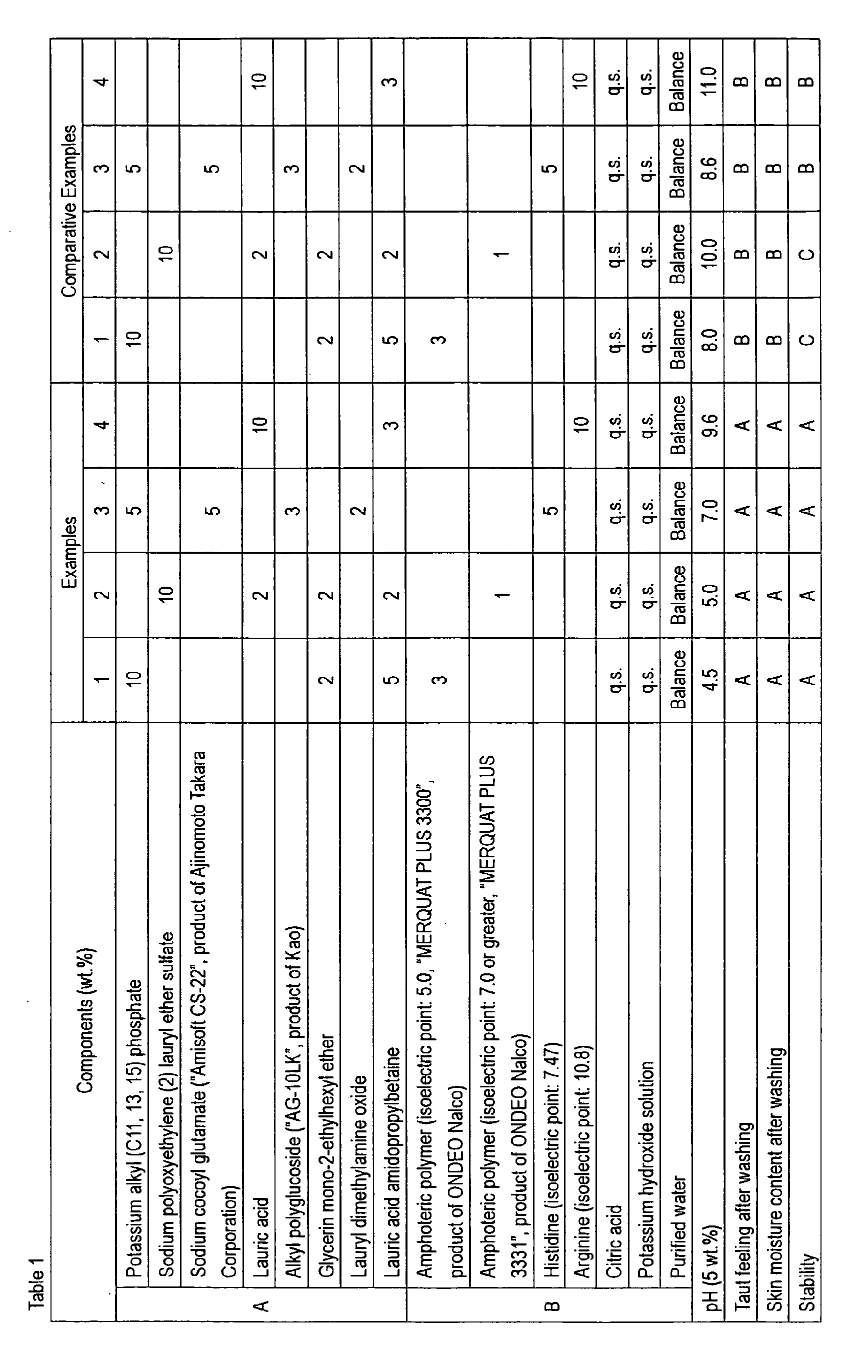

### Example 5 (Face wash)

A face wash having the below-described composition was prepared.

| (Components) | (wt.%) |
|---|---|
| Potassium alkyl (C11,13,15) phosphate | 15 |
| Glycerin mono-2-ethylhexyl ether | 5 |
| Polyoxyethylene (9) tridecyl ether | 5 |
| Cocamidopropylbetaine | 5 |
| Coconut oil fatty acid diethanolamide | 0.5 |
| Polyglyceryl monolaurate | 1 |
| Serine (isoelectric point: 5.68) | 20 |
| Carboxyvinyl polymer | 1 |
| Polyethylene oxide ("ALKOX E-100", product of Meisei Chemical Works) | 0.1 |
| Glycerin | 10 |
| Propylene glycol | 10 |
| Phosphoric acid | q.s. |
| Titanium oxide | 0.01 |
| Methylparaben | 0.2 |
| Perfume | q.s. |
| Dibutylhydroxytoluene | 0.1 |
| Purified water | Balance |
| (pH (5 wt.% aqueous solution): 5.0) | |

### Example 6 (Face wash)

A face wash having the below-described composition was prepared.

| (Components) | (Wt.%) |
|---|---|
| Sodium polyoxyethylene (2) lauryl ether phosphate | 15 |
| Sodium cocoyl glutamate ("Amisoft CS-11", product of Ajinomoto Takara Corporation) | 5 |
| Lauroyl hydroxysulfobetaine | 5 |
| Ethylene glycol distearate | 3 |
| Alanine (isoelectric point: 6.00) | 5 |
| Hydroxyethyl cellulose | 0.5 |
| Cationic polymer ("MERQUAT 550", product of ONDEO Nalco) | 0.5 |
| Sorbitol | 10 |
| Isoprene glycol | 10 |
| Sodium lactate | 1 |
| Titanium oxide coated mica ("Timiron Starluster MP-115" product of Merck Japan) | 0.01 |
| Sodium benzoate | 0.2 |
| Polyethylene beads | 0.8 |
| Perfume | q.s. |
| Talc | 3 |
| EDTA-2Na | 0.1 |
| Purified water | Balance |
| (pH (5 wt.% aqueous solution): 5.5) | |

### Example 7 (Body shampoo)

A body shampoo having the below-described composition was prepared.

| (Components) | (wt.%) |
|---|---|
| Sodium polyoxyethylene (2) lauryl ether phosphate | 20 |
| Alkyl polyglucoside ("AG-10LK", product of Kao Corporation) | 5 |
| Lauroyl hydroxysulfobetaine | 5 |
| Lauric acid diethanolamide | 3 |
| Ethylene glycol distearate | 3 |
| Arginine (isoelectric point: 10.8) | 0.5 |
| Amphoteric polymer (isoelectric point: 5.0, "MERQUAT PLUS 3330", product of ONDEO Nalco) | 3 |
| Hydroxypropyl methylcellulose | 0.5 |
| Sorbitol | 10 |
| Dipropylene glycol | 10 |
| Malic acid | q.s. |
| 1-Menthol | 0.1 |
| Sodium benzoate | 0.2 |
| Perfume | q.s. |
| 1-Hydroxyethane-1,1-diphosphonic acid ("DEQUEST 2010CS", product of Solutia Japan Ltd.) | 0.1 |
| Coloring matter (Blue No. 1) | 0.0002 |
| Purified water | Balance |
| (pH (5 wt.% aqueous solution): 5.0) | |

### Example 8 (Shampoo)

A shampoo having the below-described composition was prepared.

| (Components) | (wt.%) |
|---|---|
| Sodium polyoxyethylene (2) lauryl ether sulfate | 15 |
| Coconut oil fatty acid monoethanolamide | 5 |
| Lauric acid amidopropylbetaine | 5 |
| Cocamidopropylbetaine | 5 |
| Polyether modified silicone ("Silicone SH-3771E", product of Dow Corning Toray Silicone) | 0.5 |
| Amphoteric polymer (isoelectric point: 7.0, "MERQUAT 2001", product of ONDEO Nalco) | 5 |
| Serine (isoelectric point: 5.68) | 0.5 |
| Dimethylpolysiloxane ("Silicone SH-200C (5000cs), product of Dow Corning Toray Silicone) | 0.5 |
| Propylene glycol | 10 |
| Ethanol | 1 |
| Silicic anhydride | 0.2 |
| Methylparaben | 0.2 |
| *Thujopsis dolabrata* extract | 0.1 |
| Oxybenzone | 0.1 |
| Perfume | q.s. |
| Citric acid | q.s. |
| Purified water | Balance |
| (pH (5 wt.% aqueous solution): 5.0) | |

### Example 9 (Hand soap)

A hand soap having the below-described composition was prepared.

| (Components) | (wt.%) |
|---|---|
| Potassium lauryl phosphate | 5 |
| Coconut oil fatty acid diethanolamide | 3 |
| Polyoxyethylene (3) polyoxypropylene (7) butyl ether | 3 |
| Lauryl dimethylamine oxide | 5 |
| Cocamidopropylbetaine | 5 |
| Arginine (isoelectric point: 10.8) | 15 |
| Maltitol | 0.5 |
| Glycerin | 10 |
| Propylene glycol | 10 |
| Ethanol | 5 |
| Tuberose polysaccharide | 0.5 |
| Isopropylmethylphenol (product of Osaka Chemicals Co.) | 0.01 |
| Methylparaben | 0.2 |
| 1-Menthol | 0.1 |
| Perfume | q.s. |
| Purified water | Balance |
| (pH (5 wt.% aqueous solution): 8.5) | |

### Example 10 (Medicated hand soap)

A medicated hand soap having the below-described composition was prepared.

| (Components) | (wt.%) |
|---|---|
| Alkyl glucoside ("AG-10LK", product of Kao Corporation) | 10 |
| Polyoxyethylene (4) lauryl ether | 3 |
| Polyoxyethylene (20) coconut oil fatty acid sorbitan ("RHEODOL TW-L120", product of Kao Corporation) | 1 |
| Polyoxyethylene (60) hydrogenated castor oil | 0.5 |
| Cationic bactericide ("SANISOL P", product of Kao Corporation) | 1 |
| Lauryltrimethylammonium chloride | 10 |
| Serine (isoelectric point: 5.68) | 0.5 |
| Glycerin | 10 |
| Amphoteric polymer (isoelectric point: 14 or greater, "MERQUAT 280", product of ONDEO Nalco) | 1 |
| Ethanol | 3 |
| Perfume | q.s. |
| Coloring matter (Yellow No. 1) | 0.0001 |
| EDTA-2Na | 0.1 |
| Methylparaben | 0.1 |
| Succinic acid | q.s. |
| Purified water | Balance |
| (pH (5 wt.% aqueous solution): 5.5) | |

The skin washed with each of the skin cleansing compositions obtained in Examples 5 to 7, 9 and 10 after the addition of water thereto is substantially free from a taut feeling and has a moist feeling after washing. The hair shampooed with the hair cleansing composition of Example 8 after the addition of water thereto is soft to the touch and substantially free from tangles so that fingers glide through the hair smoothly after shampooing. In addition, the cleansing compositions obtained in Examples 5 to 10 each has excellent stability.

### Industrial Applicability

The cleansing composition of the present invention has for example, excellent stability, causes substantially no taut feeling, gives the skin a moist feeling after washing with the composition, and makes the hair soft and substantially free from tangles so that fingers glide through the hair smoothly after shampooing.

## Claims

1. A cleansing composition comprising (A) a surfactant, (B) an amino acid or amphoteric polymer and (C) water, the composition having a pH not greater than an isoelectric point of component (B) when diluted to 5 wt.% with purified water.

2. A cleansing composition comprising (A) a surfactant, (B) an amino acid or amphoteric polymer and (C) water, the composition having a pH not greater than an isoelectric point of component (B) when diluted with water upon use.

3. The cleansing composition of Claim 1 or 2, wherein the amino acid as component (B) is at least one component selected from the group consisting of glycine, alanine, serine, arginine, histidine, threonine, asparagine, glutamine, leucine, valine, lysine, phenylalanine, tyrosine, urocanic acid, proline, oxyproline, isoleucine, cysteine, methionine, tryptophan, glutamic acid and aspartic acid.

4. The cleansing composition of Claim 1 or 2, wherein the amphoteric polymer as component (B) is at least one component selected from the group consisting of acrylic acid/dimethyldiallylammonium chloride copolymer, acrylamide/acrylic acid/dimethyldiallylammonium chloride copolymer, methyl acrylate/acrylic acid/methacrylamidopropyltrimethylammonium chloride copolymer and acrylamide/acrylic acid/methacrylamidopropyltrimethylammonium chloride copolymer.

5. The cleansing. composition of Claim 1 or 2, wherein component (B) has an isoelectric point of from 3 to 12.

6. The cleansing composition of Claim 1 or 2, wherein component (A) is an anionic surfactant.

7. The cleansing composition of Claim 1 or 2, wherein component (A) and component (B) are incorporated in amounts of from 1 to 50 wt.% and from 0.1 to 30 wt.%, respectively, by weight of the composition.

8. The cleansing composition of Claim 1 or 2, which is a skin or hair cleansing composition.

9. A body cleansing method, which comprises adding water to a cleansing composition containing (A) a surfactant, (B) an amino acid or amphoteric polymer and water, the composition having a pH not greater than an isoelectric point of component (B) when diluted to 5 wt.% with purified water, and washing a body with the mixture.
